(19) Europäisches Patentamt
European Patent Office
Office européen des brevets

(11) Numéro de publication: **0 007 823**
**B1**

# (12) FASCICULE DE BREVET EUROPEEN

(45) Date de publication du fascicule du brevet: **20.07.83**

(21) Numéro de dépôt: **79400426.7**

(22) Date de dépôt: **27.06.79**

(51) Int. Cl.³: **C 07 J 9/00**, A 61 K 31/575, C 07 B 23/00, A 61 K 49/02 // C07J21/00

(54) **Nouveaux dérivés 3,20-dioxo 4,9-diène 21-hydroxyle stéroides, leur procédé de préparation, leur application à titre de médicaments et les compositions pharmaceutiques les renfermant.**

(30) Priorité: **13.07.78 FR 7820973**

(43) Date de publication de la demande: **06.02.80 Bulletin 80/3**

(45) Mention de la délivrance du brevet: **20.07.83 Bulletin 83/29**

(84) Etats contractants désignés: **AT BE CH DE FR GB IT NL SE**

(56) Documents cités:
**FR-A-2 077 877**
**FR-A-2 149 302**
**FR-A-2 374 335**
**FR-A-2 385 737**
**GB-A- 902 373**
***STEROIDS*, vol. 22 (1), juillet 1973 San Francisco, US, DANIEL PHILIBERT et al.: «Progesterone binding in the immature mouse and rat uterus», pages 89–98**

(73) Titulaire: **ROUSSEL-UCLAF, 102, route de Noisy Boîte postale no.9, F-93230 Romainville (FR)**

(72) Inventeur: **Coussediere, Daniel, 134, rue de la Fontaine Jean Valjean, F-93370 Montfermeil (FR)**

(74) Mandataire: **Tonnellier, Marie-José et al, 102, route de Noisy Boîte postale no 9, F-93230 Romainville (FR)**

**EP 0 007 823 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Nouveaux dérivés 3, 20-dioxo 4,9-diène 21-hydroxyle stéroïdes, leur procédé de préparation, leur application à titre de médicaments et les compositions pharmaceutiques les renfermant

La présente invention concerne de nouveaux dérivés 3,20-dioxo 4,9-diène 21-hydroxyle stéroïdes, leur procédé de préparation, leur application comme médicaments et les compositions pharmaceutiques les renfermant.

Dans le brevet français 2072877 est décrit le 3,20-dioxo 17α, 21-diméthyl 19-nor pregna 4,9-diène. Ce produit diffère des produits de la présente demande en ce qu'il ne comporte pas de radicalhydroxyle en position 21.

Dans le brevet français 2149302 est décrit un procédé de préparation de produits de formule

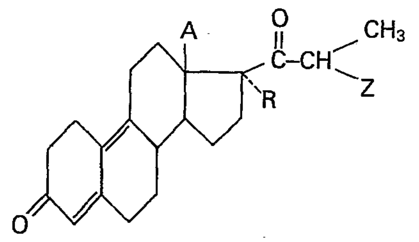

dans laquelle A alcoyle ayant de 1 à 3 atomes de carbone, Z hydrogène ou méthyle et R hydrogène, méthyle ou un groupement OR'$_1$, R'$_1$ hydrogène ou un groupement acyle. De même que le produit du brevet précédemment cité, ces produits ne comportent pas de radical hydroxyle en position 21.

Dans le brevet britannique 902273 sont décrits des produits de formule

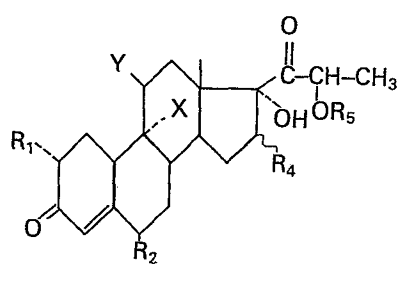

dans laquelle $R_1$ = hydrogène ou méthyle, $R_2$ = hydrogène, fluor, chlore ou méthyle, $R_3$ = hydrogène ou hydroxyle, $R_4$ = hydrogène, α-fluor, α ou β-méthyle, méthylène ou α-hydroxyle libre ou acylé, $R_5$ = hydrogène, X = hydrogène, halogène, méthoxy ou éthoxy, Y = cétone ou β-hydroxy, trois au moins de $R_1$, $R_2$, $R_3$ et $R_4$ représentent un hydrogène, ainsi que les dérivés $\Delta_1$, $\Delta_6$ et $\Delta_{1-6}$ correspondants.

En particulier à cause du substituant céto ou hydroxyle en 11 et de l'absence de double liaison en 9 (10), les produits du brevet britannique cité sont différents des produits de la présente demande.

L'invention a pour objet les composés de formule (I):

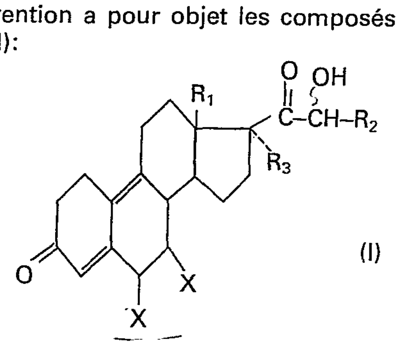

(I)

dans laquelle X représente un atome d'hydrogène ou un atome de tritium, $R_1$ représente un radical alcoyle renfermant de 1 à 3 atomes de carbone, $R_2$ un radical alcoyle renfermant de 1 à 12 atomes de carbone, $R_3$ un radical alcoyle renfermant de 1 à 4 atomes de carbone, sous forme de chacun des épimères 21R ou 21S ou sous forme d'un mélange de ces épimères.

Parmi les composés de formule I, on peut citer, en particulier, ceux pour lesquels X représente un atome d'hydrogène.

$R_1$ représente, de préférence, le radical méthyle ou éthyle.

$R_2$ représente, de préférence, le radical méthyle, éthyle, n-propyle, isopropyle, n-butyle, isobutyle, n-pentyle, n-hexyle, 2-méthylpentyle, 2,3-diméthylbutyle, n-octyle, ou 2,2-diméthyl-hexyle.

$R_3$ représente, de préférence, le radical méthyle, éthyle ou n-propyle.

L'invention a tout particulièrement pour objet les composés de formule I pour lesquels $R_1$ représente un radical méthyle, sous forme de chacun des épimères 21R ou 21S ou sous forme d'un mélange de ces épimères, ceux pour lesquels $R_2$ représente un radical méthyle, sous forme de chacun des épimères 21R ou 21S ou sous forme d'un mélange de ces épimères, ainsi que ceux pour lesquels $R_3$ représente un radical méthyle, sous forme de chacun des épimères 21R ou 21S ou sous forme d'un mélange de ces épimères.

L'invention a plus spécialement pour objet les composés dont la préparation est donnée plus loin dans la partie expérimentale, sous forme de chacun des épimères 21R ou 21S au niveau de l'hydroxyle ou sous forme d'un mélange de ces épimères et tout spécialement, les composés appelés A dans les exemples ci-après, dont l'hydroxyle en 21 est en position S.

Les composés de formule I pour lesquels X représente un atome d'hydrogène présentent d'intéressantes propriétés pharmacologiques, notamment, une activité progestomimétique et une activité anti-estrogène remarquables, comme le montrent les résultats de tests exposés ci-après dans la partie expérimentale. Ces propriétés rendent lesdits produits de formule I aptes à être utilisés comme médicaments progestatifs inhibiteurs hypophysaires à prédominance anti-LH ou comme anti-estrogènes. Ils trouvent leur emploi dans le traitement des dysménorrhées, des stérilités, des dystrophies ovariennes par mise au repos des ovaires, dans le traitement des tumeurs du sein et de l'utérus, et comme contraceptifs.

L'invention a donc pour objet lesdits produits de formule I à titre de médicaments.

L'invention a plus spécialement pour objet, à titre de médicament, les composés de formule I pour lesquels X représente un atome d'hydrogène, dont la préparation est donnée plus loin dans la partie expérimentale.

Le médicament préféré de l'invention est le composé appelé A dans l'exemple 1 ou 2 ci-après, dont l'hydroxyle en 21 est en position S.

La posologie utile varie en fonction de l'affection à traiter et de la voie d'administration; elle peut aller, par exemple, de 10 µg à 50 mg par jour, par voie orale, chez la femme adulte, pour le produit de l'exemple 1 nommé A dans la partie expérimentale. Lesdits composés de formule I sont utilisés par voie buccale, rectale, parentérale ou par voie locale en application topique sur la peau et les muqueuses. Ils peuvent être prescrits sous forme de comprimés, de comprimés enrobés, de cachets, de capsules, de granulés, d'émulsions, de sirops, de suppositoires, de solutés et de suspensions injectables, de pommades, de crèmes, de gels et de préparations en aérosols.

L'invention a donc également pour objet les compositions pharmaceutiques renfermant, comme principe actif, au moins un composé de formule I, dans laquelle X représente un atome d'hydrogène.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs. Ces compositions pharmaceutiques peuvent être préparées selon les méthodes usuelles.

Les composés de formule I, pour lesquels X représente un atome de tritium possèdent, de manière générale, les mêmes propriétés pharmacologiques que les produits de formule I pour lesquels X représente un atome d'hydrogène.

De plus, ces composés et notamment l'isomère S en 21 décrit ci-après dans la partie expérimentale, en raison de leur activité spécifique, permettent la localisation et le dosage aisé du récepteur spécifique de la progestérone, notamment dans le cytosol utérin ou dans le cytoplasme de cellules tumorales (cancer du sein) et dans les tumeurs induites par le DMBA (le 9,10-diméthyl 1,2-benzanthracène) chez le rat.

Ces produits présentent l'avantage, par rapport à la progestérone, de ne pas se fixer sur la transcortine et d'avoir une affinité pour ledit récepteur de la progestérone 2 à 10 fois supérieure à l'affinité de cette dernière.

L'invention a ainsi également pour objet l'application des composés de formule I, pour lesquels X représente un atome de tritium, à la localisation et au dosage es récepteurs spécifiques de la progestérone, notamment dans le cytosol utérin et dans le cytoplasme des cellules tumorales, ainsi que dans les tumeurs induites par le 9,10-diméthyl 1,2-benzanthracène chez le rat.

L'invention a également pour objet un moyen de dosage et de localisation des récepteurs spécifiques de la progestérone, notamment dans le cytosol utérin et dans le cytoplasme des cellules tumorales, ainsi que dans les tumeurs induites par le 9,10-diméthyl 1,2-benzanthracène chez le rat.

Les composés de formule I, dans lesquels X représente un atome de tritium, présentent une activité spécifique de l'ordre de 50 Ci/mM.

Ces composés peuvent être utilisés pour la localisation et le dosage des récepteurs spécifiques de la progestérone, selon des techniques décrites dans de nombreuses publications comme, par exemple, J.P. Raynaud and D. Philibert – Steroids – July 1973, p.p. 89–97).

L'invention a également pour objet un procédé de préparation des composés de formule I dans laquelle X représente un atome d'hydrogène, caractérisé en ce que l'on soumet un composé de formule II:

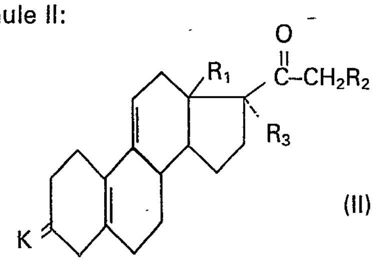

(II)

dans laquelle K représente un groupement cétal, $R_1$, $R_2$ et $R_3$ conservant la même signification que précédemment, à l'action d'un agent d'oxydation en présence d'un alcoolate tertiaire, puis à celle d'un agent de réduction pour obtenir le composé de formule III:

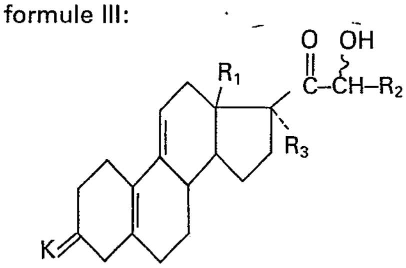

(III)

sous forme d'un mélange d'épimères 21R et 21S, que l'on soumet à l'action d'un agent d'hydrolyse acide capable d'hydrolyser le groupement cétal et d'isomériser le système de doubles liaisons $\Delta$ 5 (10) 9 (11) en un système $\Delta$ 4,9 pour obtenir le composé de formule I correspondant:

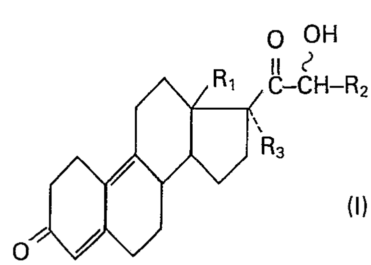

(I)

sous forme d'un mélange d'épimères 21R et 21S que l'on sépare, si désiré, en chacun des épimères.

K représente de préférence un groupement alkyl-cétal cyclique ayant de 2 à 4 atomes de carbone et, notamment, l'éthylènecétal ou le propylènecétal ou bien un dialkylcétal comme, par exemple, le diméthyl ou le diéthylcétal.

L'agent d'oxydation est, de préférence, l'oxygène moléculaire.

L'alcoolate tertiaire est, de préférence, un terbutylate ou un teramylate de métal alcalin, comme par exemple, le terbutylate ou le teramylate de sodium, de potassium ou de lithium.

L'agent de réduction utilisé est, de préférence, un trialcoyl -phosphite, par exemple, le triméthyl- ou le triéthylphosphite.

L'agent d'hydrolyse acide, capable d'hydrolyser le groupement cétal et d'isomériser le système de doubles liaisons △ 5 (10) 9 (11) en un système △ 4,9 est, de préférence, une résine sulfonique du commerce, à support de polystyrène ou à support de polymère styrène/divinyl-benzène, mais on peut également utiliser un acide minéral tel que l'acide chlorhydrique ou l'acide sulfurique; on peut également utiliser l'acide paratoluène sulfonique ou l'acide perchlorique.

Les épimères obtenus peuvent être séparés selon les méthodes classiques de chromatographie.

L'invention a également pour objet un procédé pour préparer les composés de formule I, telle que définie ci-dessus, caractérisé en ce que l'on soumet un composé de formule $II_A$:

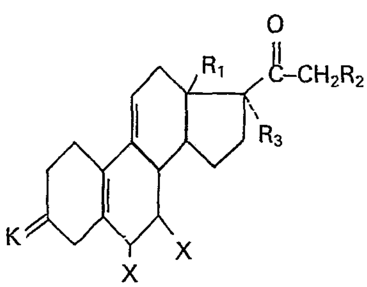

$(II_A)$

dans laquelle K, $R_1$, $R_2$, $R_3$ et X ont la signification déjà indiquée, à l'action d'une base forte pour former l'énolate intermédiaire que l'on soumet à l'action de l'iode pour obtenir le composé de formule IV:

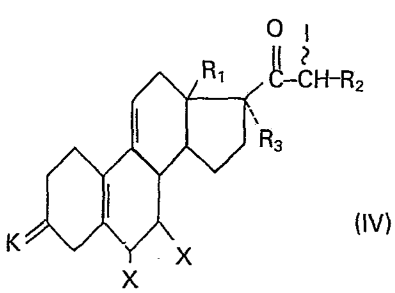

(IV)

sous forme d'un mélange d'isomères 21R et 21S, que l'on soumet à l'action d'un acétate alcalin, pour obtenir le composé de formule V:

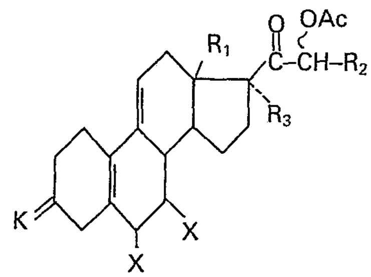

(V)

sous forme d'un mélange d'isomères 21R et 21S, que l'on soumet à l'action d'un agent d'hydrolyse acide capable d'hydrolyser le groupement cétal et d'isomériser le système de doubles liaisons △ 5 (10) 9 (11) en un système △ 4,9, pour obtenir le composé de formule VI:

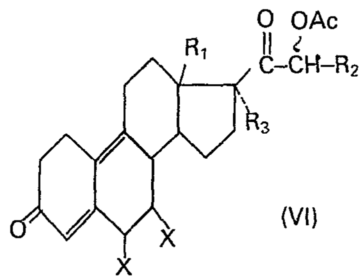

(VI)

sous forme d'un mélange d'isomère 21R et 21S, que l'on soumet à l'action d'un agent de saponification, pour obtenir le composé de formule $I_A$:

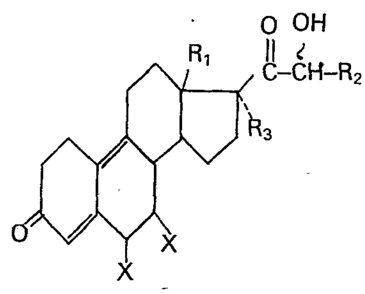

$(I_A)$

sous forme d'un mélange d'isomères 21R et 21S, que l'on sépare, si désiré, en chacun des épimères.

K a, de préférence, les valeurs indiquées précédemment.

La base forte est, de préférence, le butyl lithium et l'on opère en présence d'une amine qui est notamment la N-cyclohexyl isopropylamine. La base forte utilisée peut également être un hydrure alcalin ou un terbutylate alcalin.

L'acétate alcalin est, de préférence, l'acétate de sodium 20 ou de potassium.

L'agent d'hydrolyse acide capable d'hydrolyser le groupement cétal et d'isomériser le système de doubles liaisons △ 5 (10) 9 (11) en un système △ 4,9 peut être l'un de ceux qui ont été cités précédemment.

L'agent de saponification peut être notamment une base alcaline comme la soude ou la potasse.

Les épimères obtenus peuvent, comme ci-dessus, être séparés par les méthodes classiques de chromatographie.

Les composés de formules III, IV, V et VI, obtenus lors de la mise en œuvre du procédé de l'invention, sont des produits chimiques nouveaux.

Les composés de formule II utilisés comme produits de départ du procédé de l'invention, sont des produits connus d'une façon générale, ils peuvent être préparés par exemple selon le procédé du brevet français 2149302.

Les composés de formule $II_A$ dans laquelle X représente un atome de tritium peuvent être préparés selon le procédé de la demande française publiée n° 2374335.

Les exemples suivants illustrent l'invention sans toutefois la limiter.

Exemple 1

17α-méthyl 17β-(2-hydroxy 1-oxo propyl) estra 4,9-dien 3-one.

Stade A:

3-(1,2-éthanediyl) acétal cyclique de 17α-méthyl 17β-(2-hydroxy 1-oxopropyl) estra -5 (10), 9 (11)-dien 3-one.

On fait barboter de l'oxygène à −20°C dans une solution renfermant 907 mg de terbutylate de potassium et 0,852 $cm^3$ de triéthylphosphite dans 13,6 $cm^3$ de diméthylformamide. On ajoute lentement, à −20°C, une solution renfermant 1 g de 3-(1,2-éthandiyl) acétal cyclique de 17α-méthyl 17β-(1-oxopropyl) estra 5 (10), 9 (11) dien 3-one dans 10 $cm^3$ de diméthylformamide en maintenant toujours le barbotage d'oxygène. On laisse 30 minutes à −20°C puis l'on ajoute de l'eau et amène à pH 5,6 par addition d'acide chlorhydrique N. On extrait cinq fois par 150 $cm^3$ d'éther isopropylique. On sèche les phases organiques, les filtre et concentre à sec. On obtient ainsi 1,554 g de produit recherché que l'on utilise tel quel dans le stade suivant.

Stade B:

17α-méthyl 17β-(2-hydroxy 1-oxo propyl) estra 4,9-dien 3-one.

On ajoute 750 mg d'une résine REDEX CF dans 8 $cm^3$ d'éthanol à 95° renfermant 510 mg du produit obtenu au stade A. On chauffe au reflux le mélange obtenu pendant 7 heures. On filtre, rince la résine obtenue à l'éthanol et au chlorure de méthylène. On concentre à sec sous pression réduite. On obtient 474 mg d'une résine que l'on chromatographie sur silice: (éluant: chlorure de méthylène/acétone 9-1). On isole la fraction contenant les 2 épimères en 21. Ces deux épimères sont séparés par chromatographie liquide sous haute pression: colonnes de silice (silices 5 μ) de 6 mm de diamètre interne et de 400 mm de longueur.

Débit: 160 ml à l'heure.

Solvant: chlorure de méthylène/acétate d'éthyle (7-3).

Détecteur U.V. = 305 nm.

Le produit dont le temps de rétention est 19 minutes est appelé A.

Celui dont le temps de rétention est 17 minutes est appelé B.

Les constantes physiques du produit A sont les suivantes:

a) Spectre R.M.N. ($CDCl_3$)
$H_4$ éthylénique à 5,70 ppm
$CH_3$ $_{13}$ à 0,84 ppm
$H_{21}$ à 4,33 ppm multiplets
$CH_3$ 22 doublet à 1,27 ppm et 1,38 ppm
$CH_3$ $_{17\alpha}$ 1,18 ppm

b) Spectre de masse: pic moléculaire à 342

Les constantes physiques du produit B sont les suivantes:

a) Spectre R.M.N. ($CDCl_3$)
$H_4$ éthylénique à 5,70 ppm
$CH_3$ $_{13}$ 0,81 ppm
$H_{21}$ à 4,38 ppm, 4,50 ppm, 4,61 ppm, 4,73 ppm
$CH_3$ $_{22}$ 1,26 ppm, 1,37 ppm
$CH_3$ $_{17\alpha}$ 1,16 ppm

b) Spectre de masse pic moléculaire à 342

Exemple 2

17β-(2-hydroxy 1-oxopropyl) 17α-méthyl estra 4,9-dièn 3-one (isomère A et isomère B)

Stade A:

3-(1,2-éthanediyl) acétal cyclique de 17α-méthyl 17β-(2-iodo 1-oxopropyl) estra 5(10) 9(11)-dièn 3-one (mélange d'isomères).

a) Formation de l'énolate

On met en solution, sous atmosphère inerte, 11,1 $cm^3$ de N-cyclohexyl isopropylamine dans 145 $cm^3$ de tétrahydrofuranne, refroidit à −40°C, ajoute 23,3 $cm^3$ de butyllithium dans l'hexane, agite 5 minutes et ajoute 10,3 g de 3-(1,2-éthanediyl) acétal cyclique de 17α-méthyl 17β-(1-oxopropyl) estra 5 (10), 9 (11)-dièn 3-one en agitant encore à 40°C pendant 20 minutes.

b) Formation du dérivé iodé

On refroidit à −50°C une solution de 8,2 g d'iode dans 60 $cm^3$ de tétrahydrofuranne, ajoute en 5 minutes la solution d'énolate précédente, agite 5 minutes à −50°C et laisse la solution revenir à température ambiante. On verse celle-ci sur 100 $cm^3$ de solution saturée de chlorure d'ammonium dans l'eau, extrait à l'éther, lave avec une solution à 10% de thio sulfate de sodium dans l'eau, puis à l'eau, sèche et distille à sec sous pression réduite pour obtenir 14,5 g de produit utilisé tel quel et immédiatement pour le stade suivant.

Stade B:

3-(1,2-éthanediyl) acétal cyclique de 17α-méthyl 17β-(2-acétoxy 1-oxopropyl) estra 5(10) 9(11) dièn 3-one (mélange d'isomères).

On agite à 80°C, sous atmosphère inerte, pendant 1 heure, 14,9 g du dérivé iodé obtenu au Stade A, 29 g d'acétate de potassium anhydre et 50 $cm^3$ de diméthylformamide. On verse le mé-

lange sur 300 $cm^3$ d'eau, extrait à l'éther, lave à l'eau, sèche et concentre à sec sous pression réduite. On obtient 11,8 g de produit attendu.

Stade C:
17β-(2-acétoxy 1-oxopropyl) 17α-méthyl estra 4,9-dièn 3-one (mélange d'isomères).

On met en solution, sous atmosphère inerte, 11,8 g de produit obtenu ci-dessus, 110 $cm^3$ d'acide acétique et 11 $cm^3$ d'acide perchlorique. On agite 1 heure à température ambiante, dilue avec 400 $cm^3$ d'eau, extrait au chlorure de méthylène, lave avec une solution aqueuse saturée de bicarbonate de sodium, sèche et concentre à sec sous pression réduite. On obtient 12 g de produit que l'on purifie par chromatographie sur silice en éluant par le mélange benzène-acétate d'éthyle (8-2). On recueille 8,6 g de produit utilisé tel quel pour le stade suivant.

Stade D:
17β-(2-hydroxy 1-oxopropyl) 17α-méthyl estra 4,9-dièn 3-one (isomère A et isomère B).

On met en solution, sous atmosphère inerte, 8,6 g du produit obtenu précédemment dans 50 $cm^3$ de méthanol, ajoute 86 mg de potasse et agite 5 heures à température ambiante. On ajoute 50 $cm^3$ d'acide chlorhydrique 0,1 N et extrait au chlorure de méthylène, sèche, concentre à sec sous pression réduite et obtient 7,6 g de produit. Par chromatographie sur silice sous pression, en éluant par le mélange benzène-acétate d'éthyle (8-2), on récupère 2,7 g d'isomère B et 2,5 g d'isomère A.

On recristallise l'isomère B dans l'éther, puis dans l'éthanol et obtient 1,3 g de produit fondant à 190°C.

Analyse:
Calculé: C% 77,15, H% 8,83
Trouvé: C% 77,2, H%9,0
$/\alpha/_D^{20} = -345° \pm 4{,}5°$ (c = 1%, $CHCl_3$)

Spectre RMN ($CDCl_3$)
Hydrogène en 2 du propyle: quintuplet à 4,58 ppm J = 7
Hydrogènes en 3 du propyle: à 1,27–1,38 ppm
Hydrogènes du $CH_3$ en 17α: à 1,17 ppm
Hydrogènes du $CH_3$ en 18: à 0,82 ppm

On recristallise l'isomère A dans l'éther et obtient 1,2 g de produit fondant à 122°C.

Analyse:
Calculé: C% 77,15, H% 8,83
Trouvé: C% 77,2, H% 9,1
$/\alpha/_D^{20} = -208° \pm 3°$ (c = 1%, $CHCl_3$)

Spectre RMN ($CDCl_3$)
Hydrogène en 2 du propyle: quadruplet de 4,2 à 4,67 ppm
Hydrogènes du $CH_3$ en 17α à 1,18 ppm
Hydrogènes du $CH_3$ en 18 à 0,84 ppm

L'analyse par dichroïsme circulaire ainsi que le diagramme de diffraction des rayons X ont permis de déterminer que l'isomère appelé B ci-dessus correspond à la configuration 21R et l'isomère appelé A à la configuration 21S.

Exemple 3
/6,7 $^3H$/ 17β-(2-hydroxy 1-oxopropyl) 17α-méthyl estra 4,9-dièn 3-one (isomère A et isomère B).

Stade A:
/6,7 $^3H$/ 3-(1,2-éthanediyl) acétal cyclique de 17α-méthyl 17β-(2-iodo 1-oxopropyl) estra 5(10) 9(11) dièn 3-one (mélange d'isomères).

En opérant de la même manière qu'au Stade A de l'exemple 2 mais en partant de 17,5 mg de /6,7 $^3H$/ 3-(1,2-éthanediyl) acétal cyclique de 17α-méthyl 17β-(1-oxopropyl) estra 5(10) 9(11)-dièn 3-one, on obtient le produit attendu, sous forme de résine utilisée immédiatement pour le stade suivant.

Stade B:
/6,7 $^3H$/ 3-(1,2-éthanediyl) acétal cyclique de 17α-méthyl 17β-(2-acétoxy 1-oxopropyl) estra 5(10) 9(11) dièn 3-one (mélange d'isomères).

Le produit obtenu au stade précédent est traité comme décrit au Stade B de l'exemple 2, en extrayant par de l'acétate d'éthyle à 1‰ de triéthylamine. On obtient le produit attendu qu'on utilise tel quel pour le Stade C.

Stade C:
/6,7 $^3H$/ 17β-(2-acétoxy 1-oxopropyl) 17α-méthyl estra 4,9-dièn 3-one (mélange d'isomères).

En partant du produit obtenu précédemment, on suit la même technique qu'au Stade C de l'exemple 2, pour obtenir une résine qui est utilisée telle quelle au stade suivant.

Stade D:
/6,7 $^3H$/ 17β-(2-hydroxy 1-oxopropyl) 17α-méthyl estra 4,9-dièn 3-one (isomère A et isomère B).

On opère comme au Stade D de l'exemple 2 avec le produit tritié obtenu ci-dessus et sépare 2,64 mg d'isomère B (21R), puis 1,69 mg d'isomère A (21S).

La /6,7 $^3H$/ 3-(1,2-éthanediyl) acétal cyclique de 17α-méthyl 17β-(1-oxopropyl) estra 5(10) 9(11)-dièn 3-one utilisée au départ de l'exemple 3 a été préparée de la manière suivante:

On agite, sous atmosphère inerte, 23,8 mg de /6,7 $^3H$/ 17α-méthyl 17β-(1-oxopropyl) estra 4,9-dièn 3-one décrit dans la demande française publiée sous le n° 2374335, 2 $cm^3$ d'éthylène glycol contenant 1 mg d'acide paratoluène sulfonique et 1 $cm^3$ d'orthoformiate d'éthyle. On chauffe la suspension à 60°C pendant 10 minutes, ajoute de la triéthylamine, refroidit, ajoute une solution aqueuse saturée de bicarbonate de sodium, extrait à l'acétate d'éthyle, lave à l'eau salée, sèche et concentre à sec sous pression réduite. Le produit obtenu est purifié par chromatographie sous pression sur silice en éluant par un mélange benzène-acétate d'éthyle (9-1) contenant 5‰ de triéthylamine.

Etude pharmacologique

I. Activité progestomimétique du produit A

a) L'activité progestomimétique du produit A a été étudiée par la méthode des récepteurs hormonaux décrite par J.P. RAYNAUD et Coll. dans «J. Ster. BIOCHEM» 1975, 6, 615–622 et dans «Physiology and Genetics of Reproduction 1975 part A p. 143–160».

La technique est la suivante:

On administre à des lapines impubères 25µg d'estradiol par voie per-cutanée. Cinq jours après ce traitement, les animaux sont sacrifiés, les utérus sont alors prélevés et homogénéisés dans un tampon trométhamine 10mM, saccharose 0,25 M, HCl. pH 7,4. L'homogénat est centrifugé à 105000g pendant une heure. Le liquide surnageant ou cytosol est alors ajusté de façon à avoir une dilution de 1/50 (poids/volume).

On incube à 0°C pendant deux heures des tubes de même volume de cytosol avec une concentration fixe de 17,21-di-méthyl 19-nor-4,9-pregnadiène 3,20-dione tritié, désigné ci-après par produit R tritié en présence ou non d'une concentration croissante de 17,21-diméthyl 19-nor-4,9-pregnadiène 3,20-dione radio-inerte désignée ci-après produit R froid, de progestérone ou de produit à tester.

On détermine au bout de deux heures et au bout de 24 heures la radioactivité du produit R tritié lié par la technique d'adsorption au charbon-dextran (1,25%–0,625%).

On trace ensuite:

la droite parallèle à l'axe des abscisses, d'ordonnée

$$I_{50} = \frac{100\left(1 + \frac{Bmin}{Bo}\right)}{2}$$

Bo étant la quantité maximum de produit R tritié lié, mesurée dans l'incubat contenant uniquement du produit R tritié, Bmin étant la quantité minimum de produit R tritié lié (non spécifique), mesurée dans l'incubat contenant du produit R tritié plus un grand excès de produit R froid (2500 $10^{-9}$M)

les courbes représentant les pourcentages de produit R tritié lié $\frac{B}{Bo}$ en fonction du logarithme des concentrations du produit froid ajouté.

Les intersections de la droite $I_{50}$ et des courbes permettent de déterminer les valeurs CP et CX.

CP: concentration de la progestérone froide qui inhibe de 50% la fixation du produit R tritié.

CX: concentration du produit testé qui inhibe de 50% la fixation du produit R tritié.

L'affinité relative du produit testé ou ARL est donnée par la formule:

$$ARL = 100 \times \frac{CP}{CX}$$

Resultats:

| Produit | ARL 2 heures | ARL 24 heures |
|---|---|---|
| Progesterone | 100 | 100 |
| Produit A | 204 | 688 |

Conclusion:

Le produit A présente une affinité pour le récepteur utérin spécifique de la progesterone très supérieure à celle de la progesterone. Le produit A présente donc une très forte activité progestomimétique.

b) L'activité progestomimétique a été déterminée sur le test de CLAUBERG. Selon ce test, des groupes de trois lapines impubères sont préalablement sensibilisées par administration, par voie sous-cutanée, d'esteradiol, pendant cinq jours à la dose quotidienne de 5µg, deux jours plus tard elle sont traitées quotidiennement, pendant cinq jours, avec le médicament étudié, par voie sous-cutanée. Les animaux sont sacrifiés le sixième jour et sur les coupes de l'utérus, la prolifération en dentelle de l'endomètre, caractéristique de l'action progestomimétique, est notée en unités Mac Phail.

Le produit A est utilisé en solution dans l'huile de sésame additionnée de 5% d'alcool benzylique.

| Traitement par | Doses µg/Lapin/Jour | Unites Mac Phail |
|---|---|---|
| Produit A | 1 | 2,2 |
| | 3 | 3,4 |
| | 10 | 3,4 |

On a donc obtenu 2,2 Unités Mac Phail à la dose quotidienne de 1µg. Le produit étudié possède donc une très forte activité progestomimétique.

II. Détermination de l'activité antiestrogène du Produit A

L'activité antiestrogène de ce produit a été recherchée sur la souris impubère selon une technique inspirée du test du RUBIN, Endo. 1951, 49, 429 et voisine de celle de DORFMAN et Coll. (Méthods in Hormone Research. Dorfman, 1962, vol. II. 118).

L'estrogène utilisé est l'estradiol. Des groupes de quatre souris agées de 18 jours reçoivent, en injection souscutanée quotidienne, pendant trois jours, soit l'estradiol seul, soit le produit étudié seul, soit l'estradiol et le produit étudié. Dans ce dernier cas, les deux stéroïdes sont injectés en des points différents. Les souris sont sacrifiées le quatrième jour et leur utérus est prélevé et pesé.

L'estradiol en solution dans l'huile de sésame additionnée de 5% d'alcool benzylique, a été administré à la dose totale de 0,27µg, chaque injection étant pratiquée sous un volume de 0,1 $cm^3$/souris.

Le produit étudié a été utilisé en solution dans l'huile de sésame additionnée de 5% d'alcool benzylique et administré aux doses totales de 0,3-1-3 et 10 µg, les injections étant également pratiquées sous un volume de 0,1 $cm^3$/souris.

Les résultats obtenus sont réunis dans le tableau II.

Tableau II

| Lots | Dose totale (µg) | Moyenne des poids des utérus en mg. |
|---|---|---|
| Témoins | 0 | 14,4 |
| Estradiol | 0,27 | 99,3 |
| Produit A | 0,3 | 14,6 |
| Produit A + Estradiol | 0,3 + 0,27 | 80,5 −19% |
| Produit A | 1 | 13,0 |
| Produit A + Estradiol | 1 + 0,27 | 59,6 −39% |
| Produit A | 3 | 17,1 |
| Produit A + Estradiol | 3 + 0,27 | 38,5 −61% |
| Produit A | 10 | 15,8 |
| Produit A + Estradiol | 10 + 0,27 | 39,6 −60% |

Ces résultats montrent donc que le produit étudié possède une nette activité antiestrogène vis-à-vis de l'estradiol à la dose de 1 µg.

Exemple de compositions pharmaceutiques

On a préparé des comprimés répondant à la formule suivante:

produit A (exemple 1) 100 µg

excipient q.s. pour un comprimé terminé à 100 µg

détail de l'excipient (talc, amidon, stéarate de magnesium).

**Revendications**

1. Les composés de formule I:

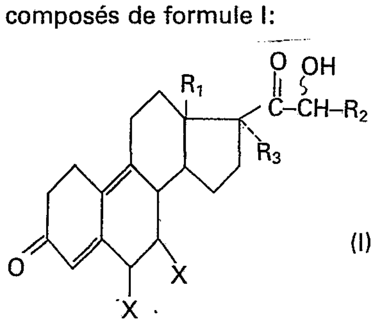

(I)

dans laquelle X représente un atome d'hydrogène ou un atome de tritium, $R_1$ représente un radical alcoyle renfermant de 1 à 3 atomes de carbone, $R_2$ un radical alcoyle renfermant de 1 à 12 atomes de carbone, $R_3$ un radical alcoyle renfermant de 1 à 4 atomes de carbone, sous forme de chacun des épimères 21R ou 21S ou sous forme d'un mélange de ces épimères.

2. Les composés de formule I, telle que définie à la revendication 1 dans laquelle X représente un atome d'hydrogène.

3. Les composés de formule I tels que définis à la revendication 1 ou 2, pour lesquels $R_1$ représente un radical méthyle, sous forme de chacun des épimères 21R ou 21S ou sous forme d'un mélange de ces épimères.

4. Les composés de formule I tels que définis à la revendication 1, 2 ou 3, pour lesquels $R_2$ représente un radical méthyle, sous forme de chacun des épimères 21R ou 21S ou sous forme d'un mélange de ces épimères.

5. Les composés de formule I, tels que définis à l'une quelconque des revendications 1 à 4, pour lesquels $R_3$ représente un radical méthyle, sous forme de chacun des épimères 21R ou 21S ou sous forme d'un mélange de ces épimères.

6. La 17α-méthyl 17β-(2-hydroxy 1-oxopropyl) estra 4,9-dièn 3-one, sous forme de chacun de ses épimères au niveau de l'hydroxyle ou d'un mélange de ses épimères.

7. La /6,7$^3$H/ 17α-méthyl 17β-(2-hydroxy 1-oxopropyl) estra 4,9-dièn 3-one, sous forme de chacun de ses épimères au niveau de l'hydroxyle ou d'un mélange de ses épimères.

8. A titre de médicament, les composés de formule I définis à l'une quelconque des revendications 1 à 5, dans laquelle X représente un atome d'hydrogène.

9. A titre de médicament, le composé défini à la revendication 6.

10. Les compositions pharmaceutiques renfermant, comme principe actif, au moins un médicament défini à la revendication 8 ou 9.

11. Application des composés de formule I, tels que définis à l'une quelconque des revendications 1, 3, 4, 5 ou 7 dans laquelle X représente un atome de tritium, à la localisation et au dosage des récepteurs spécifiques de la progestérone.

12. Moyen de dosage et de localisation des récepteurs spécifiques de la progestérone, comportant un composé de formule I, tels que définis à l'une quelconque des revendications 1, 3, 4, 5 ou 7, dans laquelle X représente un atome de tritium.

13. Procédé de préparation des composés de formule I, tels que définis à l'une quelconque des revendications 1 à 6, dans lesquels X représente un atome d'hydrogène, caractérisé en ce que l'on soumet un composé de formule II:

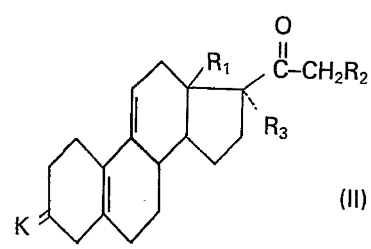

(II)

dans laquelle K représente un groupement cétal, $R_1$, $R_2$ et $R_3$ conservant la même signification que dans la revendication 1, à l'action d'un agent d'oxydation en présence d'un alcoolate tertiaire, puis à celle d'un agent de réduction pour obtenir le composé de formule III:

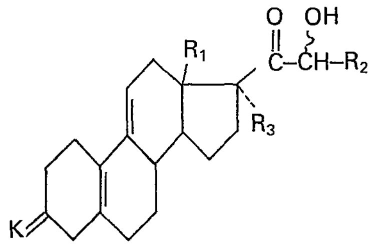

(III)

sous forme d'un mélange d'épimères 21R et 21S, que l'on soumet à l'action d'un agent d'hydrolyse acide capable d'hydrolyser le groupement cétal et d'isomériser le système de doubles liaisons △ 5(10) 9(11) en un système △ 4,9 pour obtenir le composé de formule I correspondant:

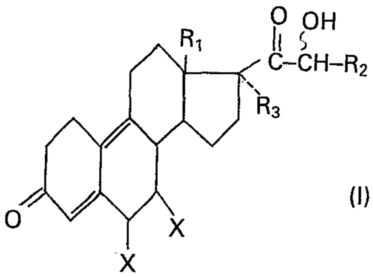

(I)

sous forme d'un mélange d'épimères 21R et 21S que l'on sépare, si désiré, en chacun des épimères.

14. Procédé de préparation des produits de formule I, tels que définis à l'une quelconque des revendications 1 à 7, caractérisé en ce que l'on soumet un composé de formule $II_A$:

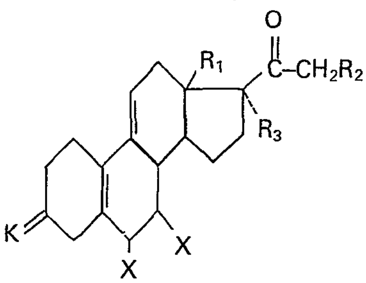

($II_A$)

dans laquelle K, $R_1$, $R_2$ et $R_3$ ont la signification indiquée à la revendication 13 et X a la signification indiquée à la revendication 1, à l'action d'une base forte pour former l'énolate intermédiaire que l'on soumet à l'action de l'iode, pour obtenir le composé de formule IV:

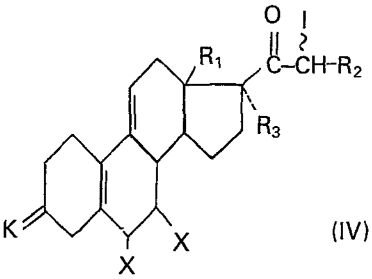

(IV)

sous forme d'un mélange d'isomères 21R et 21S, que l'on soumet à l'action d'un acétate alcalin, pour obtenir le composé de formule V:

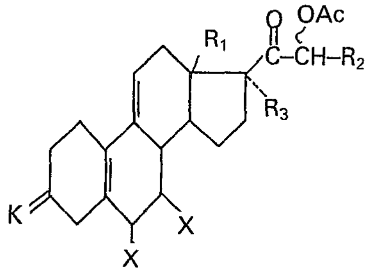

(V)

sous forme d'un mélange d'isomères 21R et 21S, que l'on soumet à l'action d'un agent d'hydrolyse acide capable d'hydrolyser le groupement cétal et d'isomériser le système de doubles liaisons △ 5(10) 9 (11) en un système △ 4,9, pour obtenir le composé de formule VI:

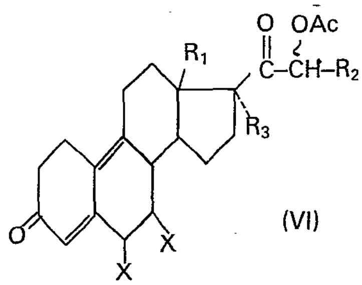

(VI)

sous forme d'un mélange d'isomères 21R et 21S, que l'on soumet à l'action d'un agent de saponification, pour obtenir le composé de formule $I_A$:

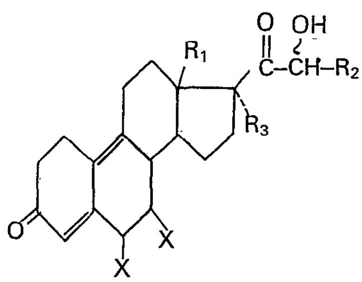

($I_A$)

sous forme d'un mélange d'isomères 21R et 21S, que l'on sépare, si désiré, en chacun des épimères.

**Patentansprüche**

1. Verbindungen der Formel I

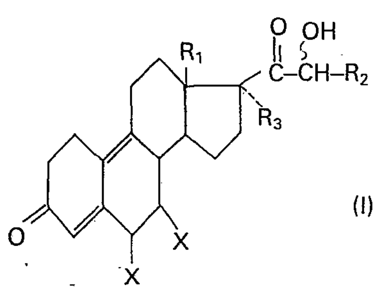

(I)

worin X ein Wasserstoffatom oder ein Tritiumatom bedeutet, $R_1$ einen Alkylrest mit 1 bis 3

Kohlenstoffatomen bedeutet, $R_2$ einen Alkylrest mit 1 bis 12 Kohlenstoffatomen bedeutet, $R_3$ einen Alkylrest mit 1 bis 4 Kohlenstoffatomen bedeutet, in Form eines jeden der 21R- oder 21S-Epimeren oder in Form eines Gemisches dieser Epimeren.

2. Verbindungen der Formel I gemäss Anspruch 1, worin X ein Wasserstoffatom bedeutet.

3. Verbindungen der Formel I gemäss Anspruch 1 oder 2, worin $R_1$ einen Methylrest bedeutet, in Form eines jeden der 21R- oder 21S-Epimeren oder in Form eines Gemisches dieser Epimeren.

4. Verbindungen der Formel I gemäss Anspruch 1, 2 oder 3, worin $R_2$ einen Methylrest bedeutet, in Form eines jeden der 21R- oder 21S-Epimeren oder in Form eines Gemisches dieser Epimeren.

5. Verbindungen der Formel I gemäss einem der Ansprüche 1 bis 4, worin $R_3$ einen Methylrest bedeutet, in Form eines jeden der 21R- oder 21S-Epimeren oder in Form eines Gemisches dieser Epimeren.

6. 17α-Methyl-17β-(2-hydroxy-1-oxopropyl)-östra-4,9-dien-3-on in Form eines jeden seiner Epimeren im Hinblick auf die Hydroxylgruppe oder in Form eines Gemisches seiner Epimeren.

7. [6,7$^3$H]-17α-Methyl-17β-(2-hydroxy-1-oxopropyl)-östra-4,9-dien-3-on in Form eines jeden seiner Epimeren im Hinblick auf die Hydroxylgruppe oder in Form eines Gemisches seiner Epimeren.

8. Als Arzneimittel die Verbindungen der Formel I gemäss einem der Ansprüche 1 bis 5, worin X ein Wasserstoffatom bedeutet.

9. Als Arzneimittel die Verbindungen gemäss Anspruch 6.

10. Pharmazeutische Zusammensetzungen enthaltend als Wirkstoff zumindest ein Arzneimittel gemäss Anspruch 8 oder 9.

11. Verwendung der Verbindungen der Formel I gemäss einem der Ansprüche 1, 3, 4, 5 oder 7, worin X ein Tritiumatom bedeutet, zur Lokalisierung und Bestimmung spezifischer Rezeptoren des Progesterons.

12. Mittel zur Bestimmung und Lokalisierung der spezifischen Rezeptoren des Progesterons, umfassend eine Verbindung der Formel I gemäss einem der Ansprüche 1, 3, 4, 5 oder 7, worin X ein Tritiumatom bedeutet.

13. Verfahren zur Herstellung von Verbindungen der Formel I gemäss einem der Ansprüche 1 bis 6, worin X ein Wasserstoffatom bedeutet, dadurch gekennzeichnet, dass man eine Verbindung der Formel II

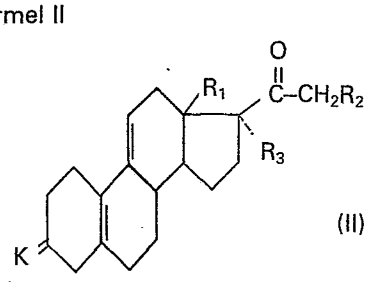

(II)

worin K eine Ketalgruppe bedeutet, $R_1$, $R_2$ und $R_3$ die in Anspruch 1 angegebene Bedeutung besitzen, der Einwirkung eines Oxidationsmittels in Gegenwart eines tertiären Alkoholats und danach derjenigen eines Reduktionsmittels unterzieht, um die Verbindung der Formel III

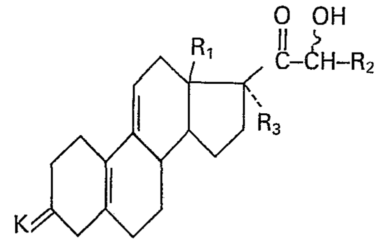

(III)

in Form eines Gemisches der 21R- und 21S-Epimeren zu erhalten, welches man der Einwirkung eines sauren Hydrolysemittels, das zur Hydrolyse der Ketalgruppe und zur Isomerisierung des △ 5(10), 9(11)-Doppelbindungssystems in ein △ 4,9-System befähigt ist, unterzieht, um die entsprechende Verbindung der Formel I

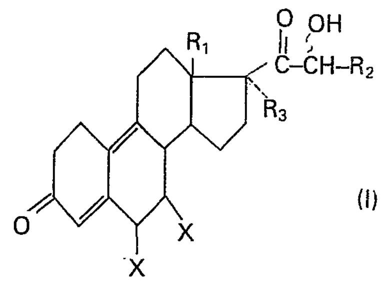

(I)

in Form eines Gemisches der 21R- und 21S-Epimeren zu erhalten, das man gewünschtenfalls in ein jedes der Epimeren auftrennt.

14. Verfahren zur Herstellung der Produkte der Formel I gemäss einem der Ansprüche 1 bis 7, dadurch gekennzeichnet, dass man eine Verbindung der Formel $II_A$

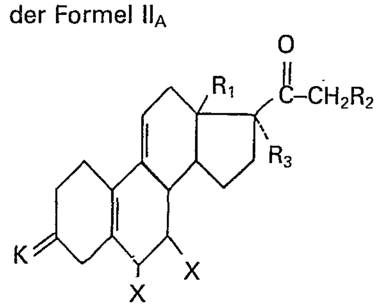

($II_A$)

worin K, $R_1$, $R_2$ und $R_3$ die in Anspruch 13 angegebene Bedeutung besitzen und X die in Anspruch 1 angegebene Bedeutung besitzt, der Einwirkung einer starken Base unterzieht, um als Zwischenprodukt das Enolat zu bilden, das man der Einwirkung von Jod unterzieht, um die Verbindung der Formel IV

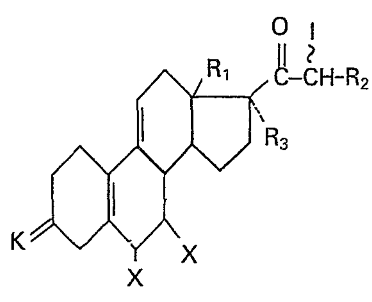

(IV)

in Form eines Gemisches der 21R- und 21S-Epimeren zu erhalten, das man der Einwirkung eines Alkaliacetats unterzieht, um die Verbindung der Formel V

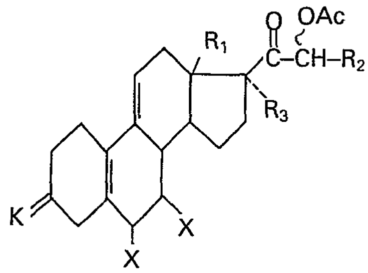

(V)

in Form eines Gemisches der 21R- und 21S-Isomeren zu erhalten, welches man der Einwirkung eines sauren Hydrolysemittels, das zur Hydrolyse der Ketalgruppe und zur Isomerisierung des △ 5(10), 9(11)-Doppelbindungssystems in ein △ 4,9-System befähigt ist, unterzieht, um die Verbindung der Formel VI

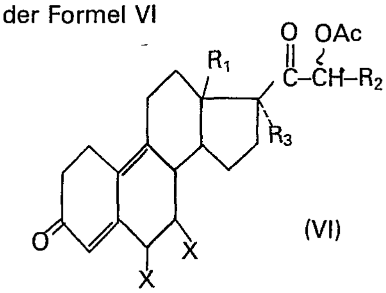

(VI)

in Form eines Gemisches der 21R- und 21S-Isomeren zu erhalten, welches man der Einwirkung eines Verseifungsmittels unterzieht, um die Verbindung der Formel $I_A$

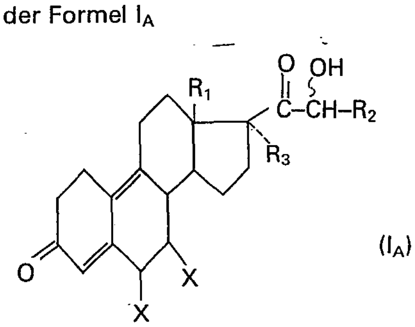

($I_A$)

in Form eines Gemisches der 21R- und 21S-Isomeren zu erhalten, das man gewünschtenfalls in ein jedes der Epimeren auftrennt.

## Claims

1. Compounds with the formula I:

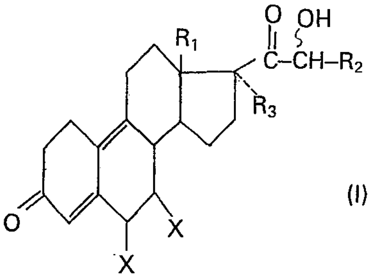

(I)

in which X represents a hydrogen atom or a tritium atom, $R_1$ represents an alkyl radical containing from 1 to 3 carbon atoms, $R_2$ an alkyl radical containing from 1 to 12 carbon atoms, $R_3$ an alkyl radical containing from 1 to 4 carbon atoms, in the form of each of the epimers 21R or 21S or in the form of a mixture of these epimers.

2. The compounds with the formula I, as defined in claim 1, in which X represents a hydrogen atom.

3. The compounds with the formula I as defined in claim 1 or 2, for which $R_1$ represents a methyl radical, in the form of each of the epimers 21R or 21S or in the form of a mixture of these epimers.

4. The compounds with the formula I as defined in claim 1, 2 or 3, for which $R_2$ represents a methyl radical, in the form of each of the epimers 21R or 21S or in the form of a mixture of these epimers.

5. The compounds with the formula I, as defined in any one of the claims 1 to 4, for which $R_3$ represents a methyl radical, in the form of each of the epimers 21R or 21S or in the form of a mixture of these epimers.

6. The 17α-methyl 17β-(2-hydroxy 1-oxopropyl) estra 4,9-dien 3-one, in the form of each of its epimers at the level of the hydroxyl or of a mixture of its epimers.

7. The [6,$7^3$H] 17α-methyl 17β-(2-hydroxy 1-oxopropyl) estra 4,9-dien 3-one, in the form of each of its epimers at the level of the hydroxyl or of a mixture of its epimers.

8. As medicament, the compounds with the formula I defined in any one of the claims 1 to 5, in which X represents a hydrogen atom.

9. As medicament, the compound defined in claim 6.

10. The pharmaceutical compositions containing, as active principle, at least one medicament defined in claim 8 or 9.

11. Application of the compounds with the formula I, as defined in any one of the claims 1, 3, 4, 5 or 7 in which X represents a tritium atom, to the localisation and to the dosage of the specific receptors of progesterone.

12. Means of dosage and of localisation of the specific receptors of progesterone, including a compound with the formula I as defined in any one of the claims 1, 3, 4, 5 or 7, in which X represents a tritium atom.

13. Process for the preparation of the compounds with the formula I, as defined in any one of the claims 1 to 6, in which X represents a hydrogen atom, characterized in that a compound with the formula II:

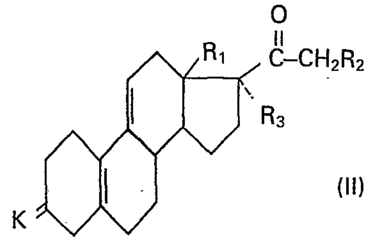

(II)

in which: X represents a ketal group, $R_1$, $R_2$ and $R_3$ retain the same significance as in claim 1, is submitted to the action of an oxidizing agent in the presence of a tertiary alcoholate, then to that of a reducing agent, so as to obtain the compound with the formula III:

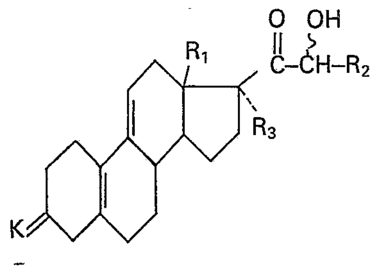

(III)

in the form of a mixture of epimers 21R and 21S, which is submitted to the action of an acid hydrolyzing agent capable of hydrolyzing the ketal group and of isomerizing the system of double bonds △ 5(10) 9(11) into a system △ 4,9 so as to obtain the corresponding compound with the formula I:

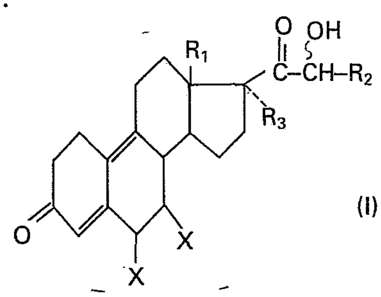

(I)

in the form of a mixture of 21R and 21S epimers, which, if desired, is separated into each of its epimers.

14. Process for the preparation of the products with the formula I, as defined in any one of the claims 1 to 7, characterized in that a compound with the formula $II_A$:

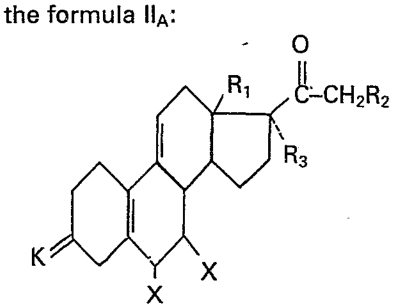

($II_A$)

in which K, $R_1$, $R_2$ and $R_3$ have the significance indicated in claim 13 and X has the significance indicated in claim 1, is submitted to the action of a strong base so as to form the intermediary enolate, which is submitted to the action of iodine, so as to obtain the compound with the formula IV:

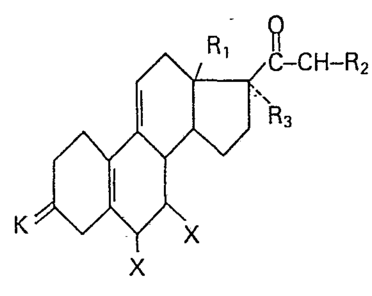

(IV)

in the form of a mixture of 21R and 21S isomers, which is submitted to the action of an alkaline acetate, so as to obtain the compound with the formula V:

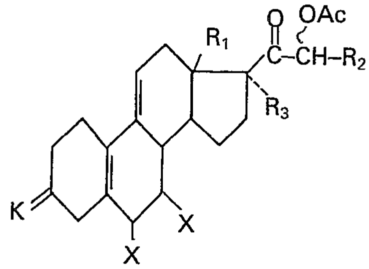

(V)

in the form of a mixture of 21R and 21S isomers, which is submitted to the acton of an acid hydrolyzing agent capable of hydrolyzing the ketal group and of isomerizing the system of double bonds △ 5(10) 9(11) into a system △ 4,9, so as to obtain the compound with the formula VI:

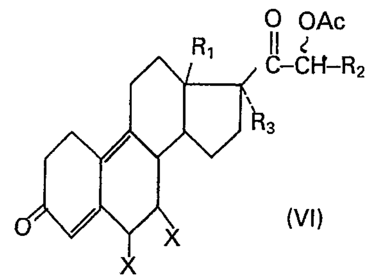

(VI)

in the form of a mixture of 21R and 21S isomers, which is submitted to the action of a saponification agent so as to obtain the compound with the formula $I_A$:

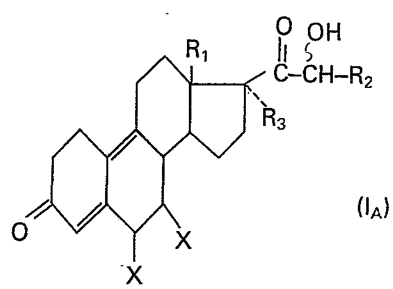

($I_A$)

in the form of a mixture of 21R and 21S isomers, which, if desired, is separated into each of its epimers.